# EUROPEAN PATENT APPLICATION

(11) **EP 4 501 321 A1**
(43) Date of publication of application: **05.02.2025**
(21) Application number: 23779426.8
(22) Date of filing: 10.03.2023
(51) Int. Cl.: A61K 31/085, A61P 13/12, A61P 39/06, A61P 43/00

(54) **NEPHROPROTECTIVE USEFUL AGENT**

(30) Priority: 29.03.2022 JP 2022053125
(71) Applicant: Watanabe Oyster Laboratory Co., Ltd., Hachioji-Shi Tokyo 192-0154 (JP)
(72) Inventor: WATANABE Mitsugu, Hachioji-shi, Tokyo 192-0154 (JP); CHIBA Hitoshi, Sapporo-shi, Hokkaido 060-0808 (JP); KEI Shukuhei, Sapporo-shi, Hokkaido 060-0808 (JP); KA Kin-you, Sapporo-shi, Hokkaido 060-0808 (JP); SAKURAI Toshihiro, Sapporo-shi, Hokkaido 060-0808 (JP); WATANABE Hideaki, Hachioji-shi, Tokyo 192-0154 (JP)
(74) Representative: Plougmann Vingtoft a/s
(86) International application number: PCT/JP2023/009236
(87) International publication number: WO 2023/189402

(57) **Abstract**

It is an objective of the present invention to provide respective useful agents that contain 3,5-dihydroxy-4-methoxybenzyl alcohol as an active ingredient and have a nephroprotective useful action, such as a nephroprotective useful agent, by further conducting the development of the inventions of respective useful agents containing 3,5-dihydroxy-4-methoxybenzyl alcohol as an active ingredient, which have already been acquired by the present inventors. The present invention is a nephroprotective useful agent including 3,5-dihydroxy-4-methoxybenzyl alcohol as an active ingredient, the nephroprotective useful agent having a nephroprotective useful action.

## Description

### TECHNICAL FIELD

The present invention relates to a nephroprotective useful agent, specifically a nephroprotective useful agent containing 3,5-dihydroxy-4-methoxybenzyl alcohol as an active ingredient and having a nephroprotective useful action.

### BACKGROUND ART

An oxidative stress state means a state in which oxidative and antioxidative functions are imbalanced in cells, and examples include a state in which excessive reactive oxygen species (ROS) accumulate in cells.

Then, the oxidative stress state causes aging in humans and various diseases, such as kidney disease.

Here, speaking of cells and mitochondria that reside in the cells, mitochondria are organelles in eucaryotes, are composed of double biological membranes, have specific DNA (mitochondrial DNA = mtDNA), fission, and increase.

The mtDNA is involved in life phenomena other than the synthesis of adenosine triphosphate (ATP) (that is, a nucleotide with three molecules of phosphoric acid bound to adenosine, which is a vital substance that mediates the storage, supply, and transportation of energy in a living organism and releases energy in association with hydrolysis into adenosine diphosphate (ADP)). In addition, the mtDNA is known as a place of oxygen respiration (aerobic respiration).

The mtDNA also plays an important role in cell apoptosis. Some of the mtDNA and its gene products are also localized on cell surfaces, and their mutations are specifically eliminated by the innate immune system.

In a human, hundreds or thousands of mitochondria reside in metabolically active cells in the liver, kidney, muscle, brain, and the like, accounting for about 40% of cytoplasm. On average, 300 to 400 mitochondria reside in one cell, accounting for 10% of the body weight in the entire body.

In mitochondria, using high-energy electrons and oxygen molecules, adenosine triphosphate (ATP) (that is, a nucleotide with three molecules of phosphoric acid bound to adenosine, which is a vital substance that mediates the storage, supply, and transportation of energy in a living organism and releases energy in association with hydrolysis into adenosine diphosphate (ADP)) as a source of energy is synthesized. However, when ATP is produced, the excessive reactive oxygen species (ROS) described above are produced as by-products.

Then, in a state of enhanced oxidative stress, mitochondrial functions are reduced in particular, and excessive reactive oxygen species (ROS) are produced. The reactive oxygen species (ROS) further stimulate the production of lipid peroxide by radical chains, falling into a vicious cycle. Therefore, maintaining mitochondrial functions is considered to be important in order to keep a balance between ROS production and removal.

In addition, many mitochondria reside especially in proximal tubular cells of the kidney (Bhargava P and Schnellmann RG, Nat Rev Nephrol, 13: 629-646, 2017). The reason for this is considered to be that a large amount of ATP is required to reabsorb nutrients and the like. Accordingly, mitochondria are considered to play an important role in the proximal tubular cells of the kidney.

The Pacific oyster is a bivalve belonging to the family Ostreidae in the order Pterioida, and its habitat extends over the whole region of East Asia, including Japan. The Pacific oyster is a nutritious foodstuff that contains a large amount of minerals, such as calcium and zinc, in addition to glycogen and protein.

The present inventors conducted a search and study of physiologically active substances from the Pacific oyster and searched for substances with antioxidant potential from the extract of the Pacific oyster. As a result, the present inventors discovered the epoch-making antioxidant substance 3,5-dihydroxy-4-methoxybenzyl alcohol (hereinafter referred to as DHMBA) and invented many useful agents in which DHMBA was used.

Up to the present, the activation of radical scavenging ability and the Keap1-Nrf2 pathway by DHMBA and associated expression induction of antioxidant gene clusters (such as HO-1 and NQO1) have been confirmed (Fuda H, Watanabe M, et al., Food Chem, 176: 226-33, 2015; Joko S, Watanabe M, et al., J Funct Foods, 35: 245-255, 2017).

The former is called direct antioxidant potential, and the latter is called indirect antioxidant potential. DHMBA was demonstrated to be a substance that has both of the functions. Furthermore, DHMBA was found to be less cytotoxic than existing antioxidant substances. However, it has not been confirmed so far whether or not DHMBA is useful for a nephroprotective action. Therefore, the present invention focused on mitochondria and verified and confirmed each protective useful effect and the like of DHMBA on mitochondria using human renal proximal tubular cells HK-2. Furthermore, from the above verification, it was confirmed that the present invention has useful actions, such as a nephroprotective useful action.

Patent Document 1: JP-A-2017-132753

### DISCLOSURE OF THE INVENTION

### PROBLEMS TO BE SOLVED BY THE INVENTION

It is an objective of the present invention to provide respective useful agents that contain 3,5-dihydroxy-4-methoxybenzyl alcohol as an active ingredient and have a nephroprotective useful action, such as a nephroprotective useful agent, by further conducting the development of the inventions of respective useful agents containing 3,5-dihydroxy-4-methoxybenzyl alcohol as an active ingredient, which have already been acquired by the present inventors.

### SOLUTIONS TO THE PROBLEMS

The present invention is a nephroprotective useful agent including
3,5-dihydroxy-4-methoxybenzyl alcohol as an active ingredient, the nephroprotective useful agent having a nephroprotective useful action,
alternatively, the present invention is a reduction promoter of intramitochondrial ROS in a human renal proximal tubular cell (HK-2) under oxidative stress stimulation including
3,5-dihydroxy-4-methoxybenzyl alcohol as an active ingredient, the reduction promoter having an effect of reducing intramitochondrial ROS in a human renal proximal tubular cell (HK-2) under oxidative stress stimulation,
alternatively, the present invention is an inhibitor of cytotoxicity of a human renal proximal tubular cell (HK-2) and an increasing agent of cell viability of a human renal proximal tubular cell (HK-2) including
3,5-dihydroxy-4-methoxybenzyl alcohol as an active ingredient, the inhibitor having an action of inhibiting cytotoxicity of a human renal proximal tubular cell (HK-2) and the increasing agent having an action of increasing cell viability of a human renal proximal tubular cell (HK-2),
alternatively, the present invention is an inhibitor of an increase in reactive oxygen accumulated in a cell of a human renal proximal tubular cell (HK-2) including
3,5-dihydroxy-4-methoxybenzyl alcohol as an active ingredient, the inhibitor having an action of inhibiting an increase in reactive oxygen accumulated in a cell of a human renal proximal tubular cell (HK-2),
alternatively, the present invention is an inhibitor of an increase in reactive oxygen accumulated in a mitochondrion of a cell of a human renal proximal tubular cell (HK-2) including
3,5-dihydroxy-4-methoxybenzyl alcohol as an active ingredient, the inhibitor having an action of inhibiting an increase in reactive oxygen accumulated in a mitochondrion of a cell of a human renal proximal tubular cell (HK-2),
alternatively, the present invention is an activator of mitochondrial respiration in a cell of a human renal proximal tubular cell (HK-2) including
3,5-dihydroxy-4-methoxybenzyl alcohol as an active ingredient, the activator having an action of activating mitochondrial respiration in a cell of a human renal proximal tubular cell (HK-2),
alternatively, the present invention is an increase promoter of a mitochondrial count in a cell of a human renal proximal tubular cell (HK-2) including
3,5-dihydroxy-4-methoxybenzyl alcohol as an active ingredient, the increase promoter having an action of increasing a mitochondrial count in a cell of a human renal proximal tubular cell (HK-2),
alternatively, the present invention is an inhibitor of mitochondrial fragmentation and an activator of a mitochondrial biosynthesis pathway in a human renal proximal tubular cell (HK-2) including
3,5-dihydroxy-4-methoxybenzyl alcohol as an active ingredient, the inhibitor having an action of inhibiting fragmentation of intracellular mitochondria in a human renal proximal tubular cell (HK-2) and the activator having an action of activating a biosynthesis pathway of the mitochondria,
or alternatively, the present invention is an elevating agent for mitochondrial basal respiration, maximal respiration, and ATP production in a human renal proximal tubular cell (HK-2) including
3,5-dihydroxy-4-methoxybenzyl alcohol as an active ingredient, the elevating agent having an action of elevating basal respiration, maximal respiration, and ATP production of intracellular mitochondria in a human renal proximal tubular cell (HK-2).

### EFFECTS OF THE INVENTION

The present invention provides an excellent effect of being able to provide not only the inventions of respective useful agents containing 3,5-dihydroxy-4-methoxybenzyl alcohol as an active ingredient but also respective useful agents containing 3,5-dihydroxy-4-methoxybenzyl alcohol as an active ingredient and having a nephroprotective useful action, such as a nephroprotective useful agent.

### BRIEF DESCRIPTION OF THE DRAWINGS

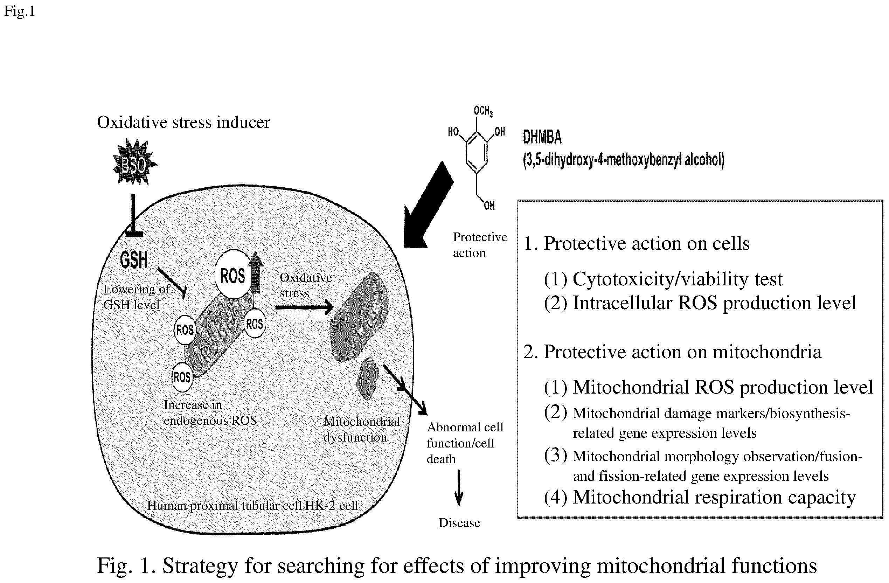

Fig. 1 is an explanatory view for describing the strategy for searching for the effects of improving mitochondrial functions.

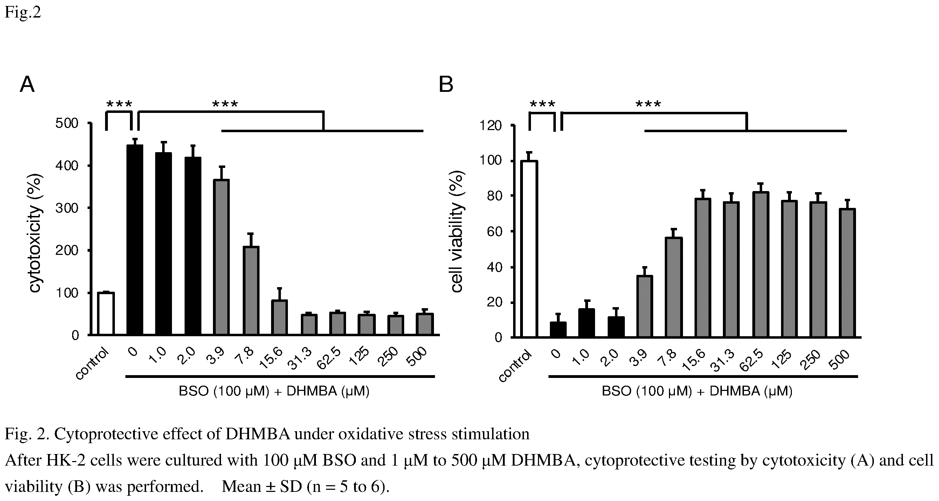

Fig. 2 is an explanatory view for describing a cytoprotective effect of DHMBA under oxidative stress stimulation. After HK-2 cells are cultured with 100 µM BSO and 1 µM to 500 µM DHMBA, cytoprotective testing by cytotoxicity (A) and cell viability (B) is performed. Mean ± SD (n = 5 to 6).

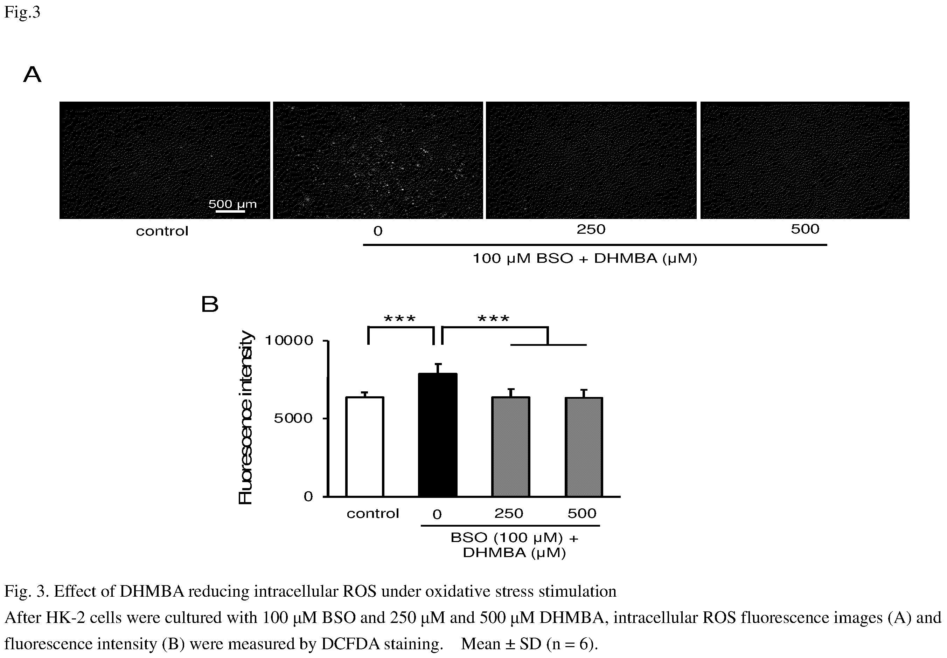

Fig. 3 is an explanatory view for describing the effect of DHMBA reducing intracellular ROS under oxidative stress stimulation. After HK-2 cells are cultured with 100 µM BSO and 250 µM and 500 µM DHMBA, intracellular ROS fluorescence images (A) and fluorescence intensity (B) are measured by DCFDA staining. Mean ± SD (n = 6).

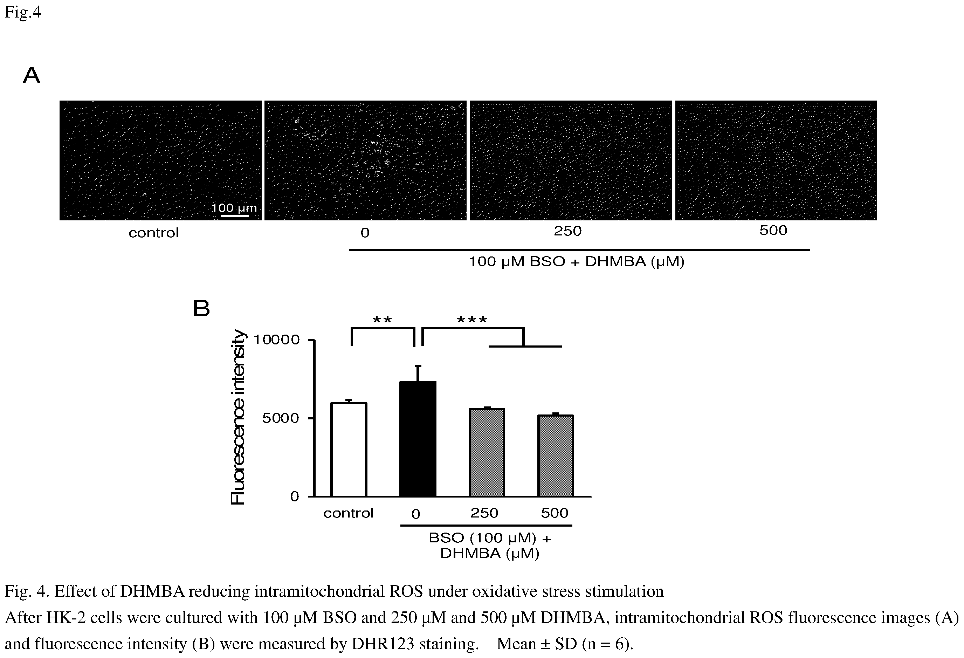

Fig. 4 is an explanatory view for describing the effect of DHMBA reducing intramitochondrial ROS under oxidative stress stimulation. After HK-2 cells are cultured with 100 µM BSO and 250 µM and 500 µM DHMBA, intramitochondrial ROS fluorescence images (A) and fluorescence intensity (B) are measured by DHR123 staining. Mean ± SD (n = 6).

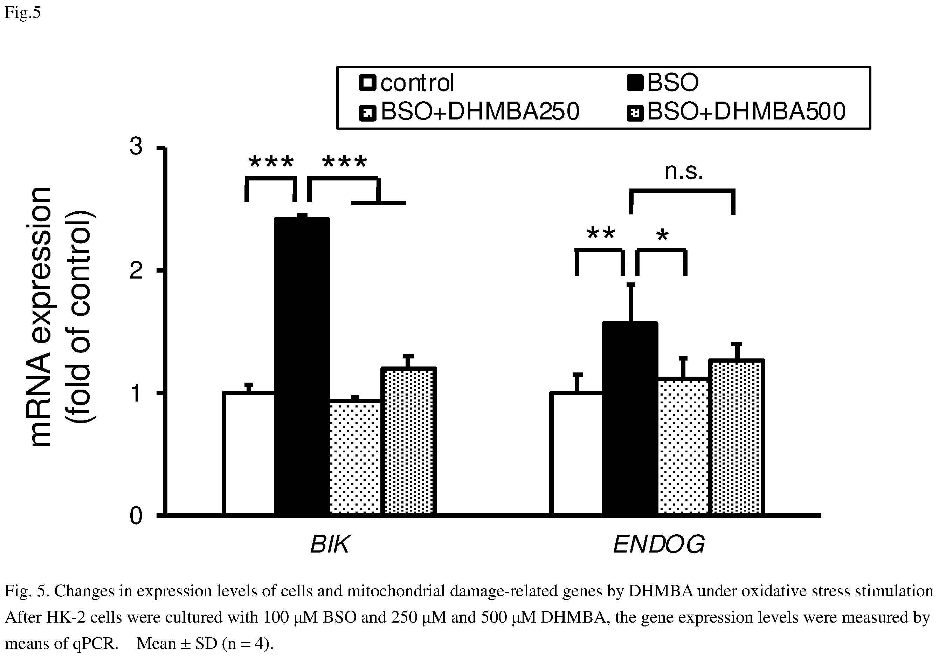

Fig. 5 is an explanatory view for describing changes in the expression levels of cells and mitochondrial damage-related genes by DHMBA under oxidative stress stimulation. After HK-2 cells are cultured with 100 µM BSO and 250 µM and 500 µM DHMBA, the gene expression levels are measured by means of qPCR. Mean ± SD (n = 4).

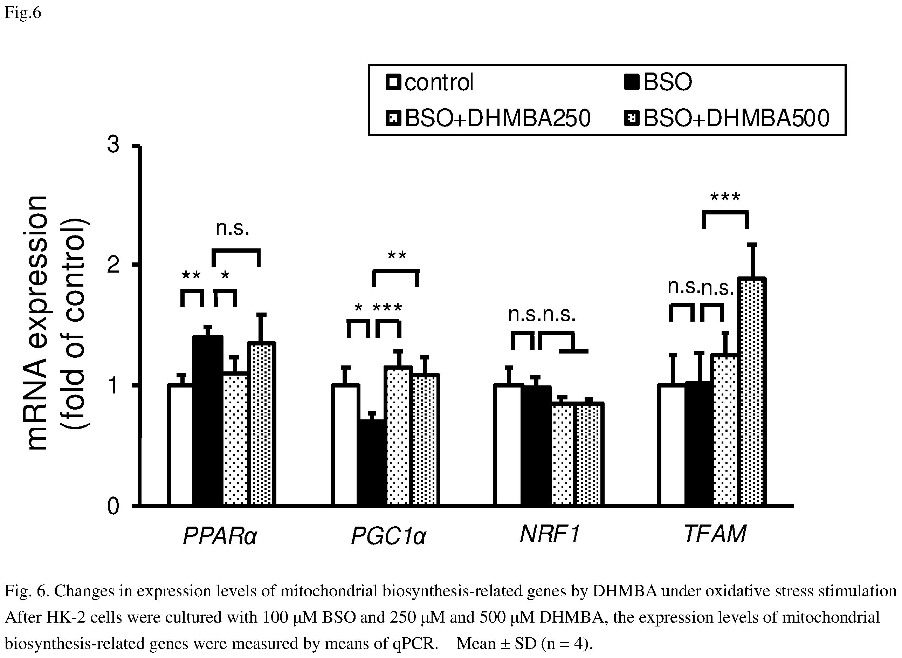

Fig. 6 is an explanatory view for describing changes in the expression levels of mitochondrial biosynthesis-related genes by DHMBA under oxidative stress stimulation. After HK-2 cells are cultured with 100 µM BSO and 250 µM and 500 µM DHMBA, the expression levels of mitochondrial biosynthesis-related genes are measured by means of qPCR. Mean ± SD (n = 4).

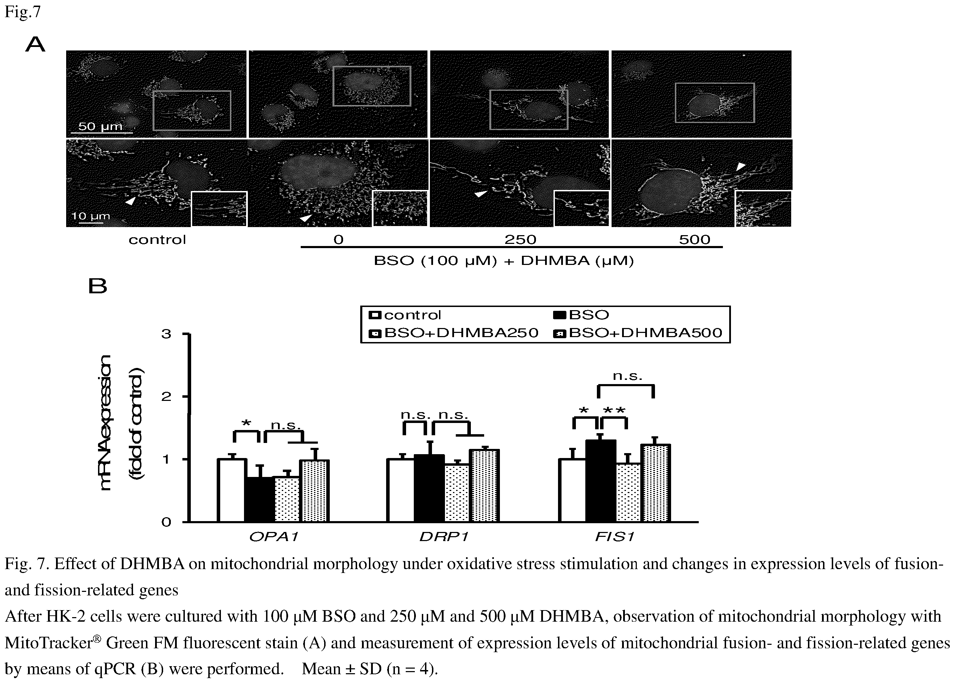

Fig. 7 is an explanatory view for describing the effect of DHMBA on mitochondrial morphology under oxidative stress stimulation and changes in the expression levels of fusion- and fission-related genes. After HK-2 cells are cultured with 100 µM BSO and 250 µM and 500 µM DHMBA, observation of mitochondrial morphology with MitoTracker (registered trademark) Green FM fluorescent stain (A) and measurement of the expression levels of mitochondrial fusion- and fission-related genes by means of qPCR (B) are performed. Mean ± SD (n = 4).

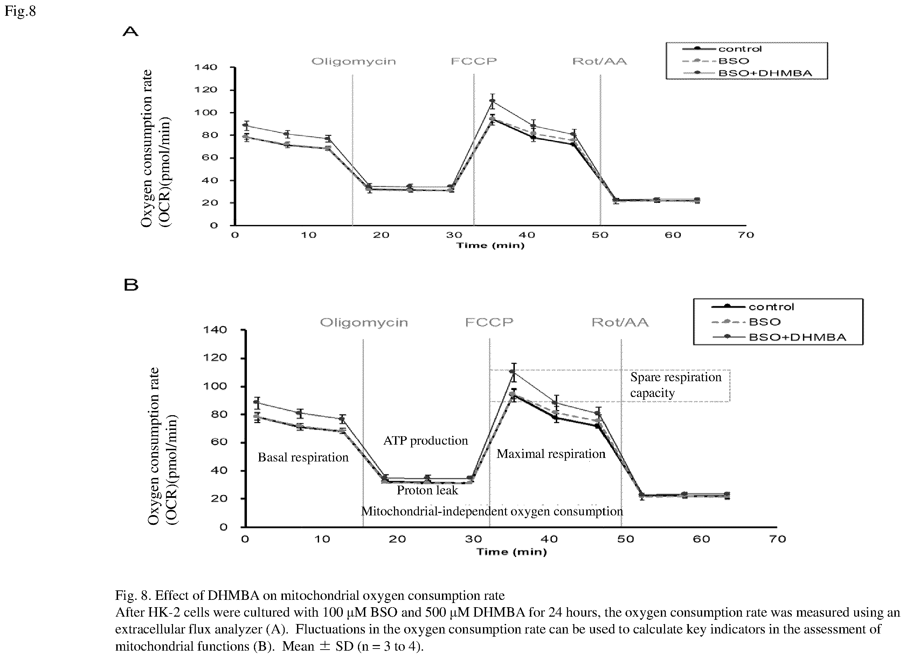

Fig. 8 is an explanatory view for describing the effect of DHMBA on the mitochondrial oxygen consumption rate. After HK-2 cells are cultured with 100 µM BSO and 500 µM DHMBA for 24 hours, the oxygen consumption rate is measured using an extracellular flux analyzer (A). Fluctuations in the oxygen consumption rate can be used to calculate key indicators in the assessment of mitochondrial functions (B). Mean ± SD (n = 3 to 4).

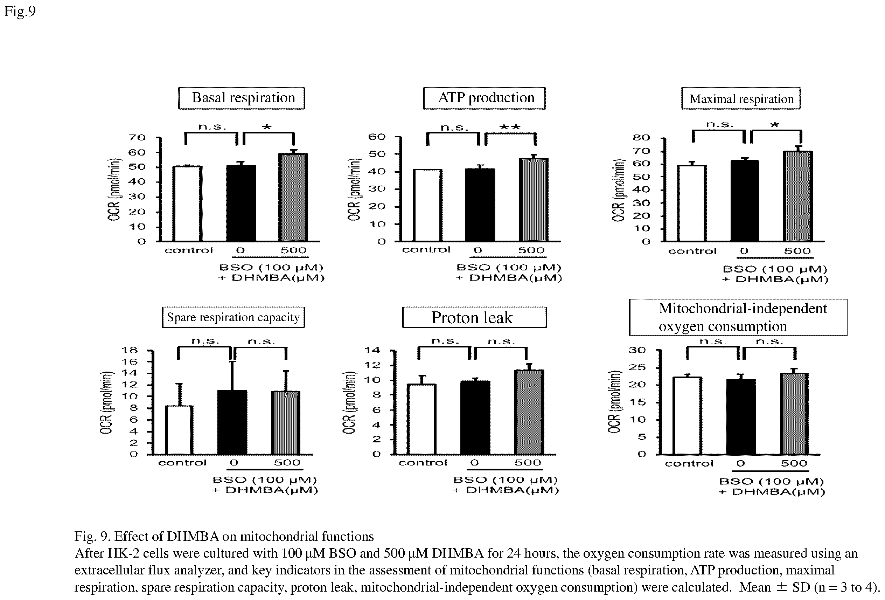

Fig. 9 is an explanatory view for describing the effect of DHMBA on mitochondrial functions. After HK-2 cells are cultured with 100 µM BSO and 500 µM DHMBA for 24 hours, the oxygen consumption rate is measured using an extracellular flux analyzer, and key indicators in the assessment of mitochondrial functions (basal respiration, ATP production, maximal respiration, spare respiration capacity, proton leak, mitochondrial-independent oxygen consumption) are calculated. Mean ± SD (n = 3 to 4).

### DESCRIPTION OF PREFERRED EMBODIMENTS

The following describes the present invention based on one example illustrated in the drawings.

### EXAMPLES

First, various kinds of verification were conducted to verify whether or not DHMBA can demonstrate usefulness to a nephroprotective action and the like. The details are described.

### "Materials and Experimental Method for Each Verification Experiment"

### (Cell culture was performed)

Human renal proximal tubular cells (HK-2) were subcultured using Dulbecco's Modified Eagle's Medium (DMEM, NACALAI TESQUE, INC.) containing 10% fetal bovine serum and 1% penicillin-streptmycin in a 5% CO₂ incubator at 37°C.

### (Cytotoxicity/viability test was performed)

In order to confirm the cytoprotective action of DHMBA on human renal proximal tubular cells HK-2 against oxidative stress, an oxidative stress inducer buthionine sulfoximine (BSO) was used to assess the toxicity and viability of the cells.

Specifically, the HK-2 cells were seeded into a 96-well plate to be 3.0 × 10³ cells/well and cultured for 24 hours.

Next, BSO and DHMBA were added in an amount of 150 µL/well to have a final concentration of BSO of 100 µM and a final concentration of DHMBA of 1 µM to 500 µM. After 48 hours of culture, 50 µL of a culture medium supernatant was taken and moved to a new 96-well plate, and a mixed liquid of a catalyst liquid of the LDH Cytotoxicity Detection Kit (Takara Bio Inc.) and a dye solution liquid (45: 1) was added in an amount of 50 µL/well. After the plate was allowed to stand for 30 minutes at room temperature, the absorbance at 490 nm was measured using a plate reader (Wallac 1420 ARVO Mx plate reader, PerkinElmer) to measure the cytotoxicity. In addition, a CCK-8 reagent (DOJINDO LABORATORIES) was added in an amount of 10 µL/well to the plate where the cells had been cultured, and after two hours of culture at 37°C, the absorbance at 450 nm was measured using a plate reader (Wallac 1420 ARVO Mx plate reader) to assess the viability of the cells. The toxicity and viability of the cells were calculated from the absorbance value relative to the control (0 µg/mL) (n = 5 to 6 for each group).

### (Intracellular ROS production level was measured)

The production level of intracellular ROS was measured to assess changes in intracellular ROS by DHMBA under oxidative stress stimulation.

The HK-2 cells were seeded into a black 96-well plate to be 3.0 × 10³ cells/well and cultured for 24 hours. Next, BSO and DHMBA were added in an amount of 100 µL/well to have a final concentration of BSO of 100 µM and a final concentration of DHMBA of 250 µM and 500 µM. After 24 hours, a supernatant was removed, a DCFDA reagent (Sigma) diluted to 25 µM with serum-free DMEM was added in an amount of 100 µL/well, and the cells were cultured at 37°C for 40 minutes. Furthermore, after the cells were washed with PBS, PBS was added in an amount of 100 µL/well, and fluorescence was measured using a plate reader (Wallac 1420 ARVO Mx plate reader) at an excitation wavelength of 485 nm and a fluorescence wavelength of 535 nm (n = 6 for each group) and observed and photographed with a fluorescence microscope BZ-9000 (Keyence).

### (Intramitochondrial ROS production level was measured)

In order to verify the effect of DHMBA on the production of intramitochondrial ROS promoted by oxidative stress stimulation, the observation and measurement of fluorescence intensity of intramitochondrial ROS were performed using DHR123.

The HK-2 cells were seeded into a black 24-well plate to be 2.0 × 10⁴ cells/well and cultured for 24 hours. Next, BSO and DHMBA were added in an amount of 500 µL/well to have a final concentration of BSO of 100 µM and a final concentration of DHMBA of 250 µM and 500 µM. After 24 hours, a supernatant was removed, a DHR123 reagent (FUJIFILM Wako Pure Chemical Corporation) diluted to 5 µM with serum-free DMEM was added in an amount of 250 µL/well, and the cells were cultured at 37°C for 40 minutes.

Furthermore, after the cells were washed with PBS, 500 µL of PBS was added, observation was performed, and photos were taken using a fluorescence microscope BZ-9000 (Keyence). Then, after the cells were peeled from the bottom with a scraper and pipetted sufficiently, an amount of 200 µL/well was moved to a black 96-well plate, and fluorescence was measured using a plate reader (Wallac 1420 ARVO Mx plate reader) at an excitation wavelength of 505 nm and a fluorescence wavelength of 534 nm (n = 6 for each group).

### (Expression levels of cells and mitochondrial damage-related genes were measured)

Under oxidative stress stimulation, ENDOG that resides in a mitochondrion migrates to the nucleus and acts on nuclear DNA fragmentation. In addition, when nuclear DNA is damaged, BIK increases and acts with intramitochondrial factors, inducing apoptosis, a type of cell death (Li LY, et al., Nature, 412: 95-99, 2001; Kutuk O, et al., PLoS One, 12: e0182809, 2017).

Therefore, in the present invention, the effect of DHMBA on cell death markers BIK and ENDOG, which increase under oxidative stress stimulation, was verified.

The HK-2 cells were seeded into a 6-well plate to be 5.0 × 10⁴ cells/well and cultured for 24 hours. Next, BSO and DHMBA were added in an amount of 2 mL/well to have a final concentration of BSO of 100 µM and a final concentration of DHMBA of 250 µM and 500 µM. After 24 hours, total RNA extraction was recovered using a NucleoSpin (registered trademark) RNA kit (MACHEREY-NAGEL). The concentration of purified RNA was quantified by the Nanodrop (Invitrogen). From 1.0 µg of total RNA, cDNA was synthesized according to the protocol of ReverTra ACE

(registered trademark) qPCR RT Master Mix with gDNA Remover (TOYOBO). The gene expression levels were measured according to the protocol of THUNDERBIRD (registered trademark) SYBR (registered trademark) qPCR Mix (TOYOBO). The measurement was performed using the CFX Connect^{™} Real-Time PCR Detection System (BioRad), and analysis was performed by the 2^{-(ΔΔCT)} method.

A comparison was performed with the gene expression level of the control group as 1. The figure was expressed as a mean value ± SD (n = 4 for each group). Each expression level was corrected by the expression level of β-actin, which is a housekeeping gene. The sequences of the primers used are shown in Table 1.

**[Table 1]**

| Table 1. Primer sequences of cells and mitochondrial damage-related genes | |
|---|---|
| Gene | Primer sequence |
| *BIK* | F: 5'- GACCTGGACCCTATGGAGGAC -3' |
| | R: 5'- CCTCAGTCTGGTCGTAGATGA -3' |
| *ENDOG* | F: 5'- AGCAGGTGGGCAAATTGAG -3' |
| | R: 5'- CCAGGATGTTTGGCACAAAGAG -3' |
| *β-actin* | F: 5'- ATAGCACAGCCTGGATAGCAACGTAC -3' |
| | R: 5'- CACCTTCTACAATGAGCTGCGTGTG -3' |

| | |
|---|---|
| F, Forward primer; R, Reverse primer. | |

### (Expression levels of mitochondrial biosynthesis-related genes were measured)

It has been reported that mitochondrial biosynthesis is regulated through activation of the PPARα/PGC1α/NRF1/TFAM pathway (Uittenbogaard M and Chiaramello A, Current pharmaceutical design, 20: 5574-5933, 2014).

The expression levels of genes related to mitochondrial biosynthesis were measured in a similar manner to that described above. A comparison was performed with the control group as 1. The figure was expressed as a mean value ± SD (n = 3 for each group). Each expression level was corrected by the expression level of β-actin. The sequences of the primers used are shown in Table 2.

**[Table 2]**

| Table 2. Primer sequences of mitochondrial biosynthesis-related genes | |
|---|---|
| Gene | Primer sequence |
| *PPAR α* | F: 5'- CGCAATCCATCGGCGAGGAT -3' |
| | R: 5'- ACCACAGGATAAGTCACCGAG -3' |
| *PGC1 α* | F: 5'- TGAACTGAGGGACAGTGATTTC -3' |
| | R: 5'- CCCAAGGGTAGCTCAGTTTATC -3' |
| *NRF1* | F: 5'- GTATCTCACCCTCCAAACCTAAC -3' |
| | R: 5'- CCAGGATCATGCTCTTGTACTT -3' |
| *TFAM* | F: 5'- ATAGGCACAGGAAACCAGTTAG -3' |
| | R: 5'- GCAGAAGTCCATGAGCTGAATA -3' |

| | |
|---|---|
| F, Forward primer; R, Reverse primer. | |

### (Mitochondrial morphology was observed)

Excessively produced ROS induces mitochondrial fission, causing mitochondrial fragmentation and dysfunction (Zorov D, et al., Cells, 8: 175, 2019). In order to observe whether or not DHMBA influences mitochondrial morphological changes under oxidative stress stimulation, double staining of mitochondria and nuclei was performed using MitoTracker (registered trademark) Green FM (Thermo Fisher Scientific), which reacts to mitochondrial membrane potential differences, and Hoechst 33342 (DOJINDO LABORATORIES), which binds to nuclear DNA.

The HK-2 cells were seeded into 35 mm dishes to be 5.0 × 10⁴ cells/dish and cultured for 24 hours. Next, BSO and DHMBA were added in an amount of 2 mL/well to have a final concentration of BSO of 100 µM and a final concentration of DHMBA of 250 µM and 500 µM. After 24 hours, a mixed solution of 0.1 µM MitoTracker (registered trademark) Green FM and 5 µg/mL Hoechst 33342 was added in an amount of 2 mL/dish, and the cells were cultured at 37°C for 20 minutes and washed with PBS. Afterwards, 1 mL of PBS was added, and fluorescence images were obtained at 100 times using a fluorescence microscope BZ-9000 (Keyence).

### (Expression levels of mitochondrial fusion- and fission-related genes were measured)

The expression levels of genes related to mitochondrial fusion (*OPA1)* and genes related to fission (*DRP1* and *FIS1*) were measured in a similar manner to that described above. A comparison was performed with the control group as 1. The figure was expressed as a mean value ± SD (n = 4 for each group). Each expression level was corrected by the expression level of β-actin. The sequences of the primers used are shown in Table 3.

**[Table 3]**

| Table 3. Primer sequences of mitochondrial fusion- and fission-related genes | |
|---|---|
| Gene | Primer sequence |
| *DRP1* | F: 5'- GGCGCTAATTCCTGTCATAA -3' |
| | R: 5'- CAGGCTTTCTAGCACTGAGC -3' |
| *FJS1* | F: 5'- AAGAGCACGCAGTTTGAGTAC -3' |
| | R: 5'- CTGGGGCTCTGTCTGCAGC -3' |
| *OPA1* | F: 5'- GGCTCCTGACACAAAGGAAA -3' |
| | R: 5'- TCCTTCCATGAGGGTCCATT -3' |

| | |
|---|---|
| F, Forward primer; R, Reverse primer. | |

### (Mitochondrial respiration capacity was measured)

The action of DHMBA improving mitochondrial respiration capacity under oxidative stress was examined using an extracellular flux analyzer XFp (Agilent Technologies).

The HK-2 cells were seeded into the XFp Cell Culture Miniplate (Agilent Technologies) to be 1.5 × 10⁴ cells/well and cultured for 24 hours. Next, BSO and DHMBA were added in an amount of 200 µL/well to have a final concentration of BSO of 100 µM and a final concentration of DHMBA of 250 µM and 500 µM. After 24 hours of culture, the culture medium was replaced with a running culture medium (XF DMEM Medium, pH 7.4, Agilent Technologies) containing 20 mM GlutaMAX (Gibco), 10 mM Pyruvate (Gibco), and glucose (10 mM), and the cells were cultured for one hour in an incubator at 37°C without CO₂ control. A 24-hour hydrated calibration plate was placed on an extracellular flux analyzer XFp and calibrated. After the calibration was completed, the calibration plate and the cell plate were exchanged, and the oxygen consumption rate (OCR) was measured. The basal respiration rate was measured first, and subsequently, oligomycin (final concentration: 2 µM) was added to inhibit ATP production. Next, FCCP (final concentration: 1 µM) was added, and the maximal respiration rate was measured. Finally, rotenon/antimycin A (final concentration: 0.25 µM) was added, and the respiration rate independent of mitochondria was measured. Each measurement was performed three times for all measurements.

### (Statistical processing was performed)

The data obtained were expressed as a mean value ± standard deviation (SD) and analyzed using JMP Pro 16 (SAS Institute Inc.). The Dunnett's test was used in multiple comparisons between groups, and *p* < 0.05 was used as the statistically significant level.

### (Results and consideration)

### (Regarding cytotoxicity and viability)

100 µM BSO (100 µM BSO + 0 µM DHMBA group) was added to the HK-2 cells, and strong cytotoxicity (Fig. 2A) and low cell viability (Fig. 2B) were observed. Next, it was observed that by the co-treatment of 100 µM BSO and DHMBA, 3.9 µM or more DHMBA inhibited cytotoxicity (Fig. 2A) and increased cell viability (Fig. 2B).

This result was considered to indicate that cell death was caused by BSO stimulation, but DHMBA inhibited it.

### (Regarding intracellular ROS production level)

An increase in intracellular ROS was observed in the cells to which BSO was added, and by the co-treatment with DHMBA, the production level of ROS decreased to the same level as that of the control group (Fig. 3A). In addition, the same result was obtained from the measured values of fluorescence intensity in Fig. 3B. Therefore, it became apparent that intracellular ROS accumulated by BSO stimulation, but DHMBA inhibited it.

Here, Fig. 3 is a drawing illustrating the effect of DHMBA reducing intracellular ROS under oxidative stress stimulation. After HK-2 cells were cultured with 100 µM BSO and 250 µM and 500 µM DHMBA, intracellular ROS fluorescence images (A) and fluorescence intensity (B) were measured by DCFDA staining.

### (Regarding intramitochondrial ROS production level)

As illustrated in Fig. 4A, ROS accumulation in mitochondria was also observed by the addition of BSO, and it was observed that the accumulation of intramitochondrial ROS was inhibited in the groups to which DHMBA was added simultaneously. In addition, the same result was obtained from the measured values of fluorescence intensity in Fig. 4B. From this result, it became apparent that intramitochondrial ROS accumulated by BSO stimulation, but DHMBA inhibited the increase.

Here, Fig. 4 is a drawing illustrating the effect of DHMBA reducing intramitochondrial ROS under oxidative stress stimulation. After HK-2 cells were cultured with 100 µM BSO and 250 µM and 500 µM DHMBA, intramitochondrial ROS fluorescence images (A) and fluorescence intensity (B) were measured by DHR123 staining. Mean ± SD (n = 6).

### (Regarding expression levels of cells and mitochondrial damage-related genes)

As illustrated in Fig. 5, the expression levels of *BIK* and *ENDOG* genes increased under oxidative stress stimulation but decreased to the same levels as those of the control group by the addition of DHMBA. From these results, it was considered that cell death was caused by BSO stimulation, but the addition of DHMBA reduced cell damage and inhibited cell death.

Here, Fig. 5 is a drawing illustrating changes in the expression levels of cells and mitochondrial damage-related genes by DHMBA under oxidative stress stimulation. After HK-2 cells were cultured with 100 µM BSO and 250 µM and 500 µM DHMBA, the gene expression levels were measured by means of qPCR. Mean ± SD (n = 4).

### (Regarding expression levels of mitochondrial biosynthesis-related genes)

As illustrated in Fig. 6, oxidative stress increased the expression level of *PPARα* genes related to mitochondrial biosynthesis but decreased the expression level of PGC1α genes. Furthermore, the addition of DHMBA increased the expression levels of *PGC1α* and *TFAM.* This result suggested that the addition of DHMBA might have activated the mitochondrial biosynthesis pathway and increased the mitochondrial count.

Here, Fig. 6 illustrates the changes in the expression level of mitochondrial biosynthesis-related genes by DHMBA under oxidative stress stimulation. After HK-2 cells were cultured with 100 µM BSO and 250 µM and 500 µM DHMBA, the expression levels of mitochondrial biosynthesis-related genes were measured by means of qPCR. Mean ± SD (n = 4).

### (Regarding mitochondrial morphology observation and measurement of fusion- and fission-related gene expression levels)

As illustrated in Fig. 7A, in the control group, it was observed that mitochondria were shaped in a long line and seemed to form a network. On the other hand, under oxidative stress stimulation, mitochondrial fragmentation was induced, the fragmented mitochondria decreased by the co-treatment with DHMBA, and mitochondrial morphology similar to that of the control group was observed. It was inferred from this result that the increase of ROS in cells and mitochondria due to the addition of BSO caused mitochondrial fragmentation, but DHMBA inhibited the fragmentation.

Furthermore, the expression levels of *OPA1* genes related to mitochondrial fusion and *DRP1* and *FIS1* genes related to fission were examined. Under oxidative stress conditions, the expression level of *OPA1* was inhibited, and the expression level of *FIS1* was enhanced (Fig. 7B). It was considered that these gene changes induced mitochondrial fragmentation, which was consistent with the results of mitochondrial morphology observation in Fig. 7A. Moreover, since the addition of DHMBA reduced the expression level of *FIS1,* it was considered that DHMBA acted on mitochondrial fragmentation in an inhibitive manner.

Here, Fig. 7 is a drawing illustrating the effect of DHMBA on mitochondrial morphology under oxidative stress stimulation and changes in the expression levels of fusion- and fission-related genes. After HK-2 cells were cultured with 100 µM BSO and 250 µM and 500 µM DHMBA, observation of mitochondrial morphology with MitoTracker (registered trademark) Green FM fluorescent stain (A) and measurement of the expression levels of mitochondrial fusion- and fission-related genes by means of qPCR (B) were performed. Mean ± SD (n = 4).

### (Regarding mitochondrial respiration capacity)

Since mitochondria play an important role in energy production, this study assessed the effect of DHMBA on mitochondrial respiration capacity. The result of measuring the oxygen consumption rate (OCR) over time with the addition of an inhibitor to the mitochondrial electron transport system is shown in Fig. 8A. In addition, fluctuations in the oxygen consumption rate by various inhibitors can be interpreted as shown in Fig. 8B. Furthermore, changes in various mitochondrial functions are shown in Fig. 9. Damage to mitochondrial functions was not recognized with the 24-hour addition of BSO. However, the addition of DHMBA increased mitochondrial basal respiration, maximal respiration, and ATP production. It was suggested that DHMBA can be expected to have an effect of increasing those mitochondrial functions.

Here, Fig. 8 is a drawing illustrating the effect of DHMBA on mitochondrial oxygen consumption rate. After HK-2 cells were cultured with 100 µM BSO and 500 µM DHMBA for 24 hours, the oxygen consumption rate was measured using an extracellular flux analyzer (Fig. 8A). Fluctuations in the oxygen consumption rate can be used to calculate key indicators in the assessment of mitochondrial functions (Fig. 8B). Mean ± SD (n = 3 to 4).

Furthermore, Fig. 9 is a drawing illustrating the effect of DHMBA on mitochondrial functions. After HK-2 cells were cultured with 100 µM BSO and 500 µM DHMBA for 24 hours, the oxygen consumption rate was measured using an extracellular flux analyzer, and key indicators in the assessment of mitochondrial functions (basal respiration, ATP production, maximal respiration, spare respiration capacity, proton leak, mitochondrial-independent oxygen consumption) were calculated. Mean ± SD (n = 3 to 4).

### (Conclusion)

In the oxidative stress state induced by the addition of BSO, the amounts of ROS in cells and mitochondria were increased to induce cell death. However, by adding DHMBA there, the ROS production level decreased, and a cytoprotective effect was observed.

In addition, it is considered that the addition of DHMBA inhibited mitochondrial fragmentation, activated the mitochondrial biosynthesis pathway, and protected mitochondria from oxidative stress damage.

Furthermore, from the result of measuring the mitochondrial respiration capacity, the mitochondrial basal respiration, ATP production, and maximal respiration increased in the oxidative stress state. These results indicated that DHMBA has a protective effect on cells and mitochondria and the action of promoting mitochondrial functions in human renal proximal tubular cells HK-2 under oxidative stress stimulation.

### (Regarding manufacturing method of the present invention)

The manufacturing method of the above-described nephroprotective useful agent and the like containing 3,5-dihydroxy-4-methoxybenzyl alcohol as an active ingredient and having a nephroprotective useful action is described.

As described above, 3,5-dihydroxy-4-methoxybenzyl alcohol can be acquired from the Pacific oyster, and the present inventors have already acquired many patents for the method of efficiently extracting and manufacturing 3,5-dihydroxy-4-methoxybenzyl alcohol from the Pacific oyster. In addition, 3,5-dihydroxy-4-methoxybenzyl alcohol can be produced by synthesis, and the present inventors have already acquired a patent for the synthesis method.

Then, in the present invention, it was assumed that 3,5-dihydroxy-4-methoxybenzyl alcohol has a lot of protective usefulness for each human organ, and experiments were conducted to verify it, leading to the invention.

Therefore, the useful agents of the present invention are manufactured after conducting these verifications.

## Claims

1. A nephroprotective useful agent comprising
3,5-dihydroxy-4-methoxybenzyl alcohol as an active ingredient, the nephroprotective useful agent having a nephroprotective useful action.

2. A reduction promoter of intramitochondrial ROS in a human renal proximal tubular cell (HK-2) under oxidative stress stimulation comprising
3,5-dihydroxy-4-methoxybenzyl alcohol as an active ingredient, the reduction promoter having an effect of reducing intramitochondrial ROS in a human renal proximal tubular cell (HK-2) under oxidative stress stimulation.

3. An inhibitor of cytotoxicity of a human renal proximal tubular cell (HK-2) and an increasing agent of cell viability of a human renal proximal tubular cell (HK-2) comprising
3,5-dihydroxy-4-methoxybenzyl alcohol as an active ingredient, the inhibitor having an action of inhibiting cytotoxicity of a human renal proximal tubular cell (HK-2) and the increasing agent having an action of increasing cell viability of a human renal proximal tubular cell (HK-2).

4. An inhibitor of an increase in reactive oxygen accumulated in a cell of a human renal proximal tubular cell (HK-2) comprising
3,5-dihydroxy-4-methoxybenzyl alcohol as an active ingredient, the inhibitor having an action of inhibiting an increase in reactive oxygen accumulated in a cell of a human renal proximal tubular cell (HK-2).

5. An inhibitor of an increase in reactive oxygen accumulated in a mitochondrion of a cell of a human renal proximal tubular cell (HK-2) comprising
3,5-dihydroxy-4-methoxybenzyl alcohol as an active ingredient, the inhibitor having an action of inhibiting an increase in reactive oxygen accumulated in a mitochondrion of a cell of a human renal proximal tubular cell (HK-2).

6. An activator of mitochondrial respiration in a cell of a human renal proximal tubular cell (HK-2) comprising
3,5-dihydroxy-4-methoxybenzyl alcohol as an active ingredient, the activator having an action of activating mitochondrial respiration in a cell of a human renal proximal tubular cell (HK-2).

7. An increasing agent of a mitochondrial count in a cell of a human renal proximal tubular cell (HK-2) comprising
3,5-dihydroxy-4-methoxybenzyl alcohol as an active ingredient, the increasing agent having an action of increasing a mitochondrial count in a cell of a human renal proximal tubular cell (HK-2).

8. An inhibitor of mitochondrial fragmentation and an activator of a mitochondrial biosynthesis pathway in a human renal proximal tubular cell (HK-2) comprising
3,5-dihydroxy-4-methoxybenzyl alcohol as an active ingredient, the inhibitor having an action of inhibiting fragmentation of intracellular mitochondria in a human renal proximal tubular cell (HK-2) and the activator having an action of activating a biosynthesis pathway of the mitochondria.

9. An elevating agent for mitochondrial basal respiration, maximal respiration, and ATP production in a human renal proximal tubular cell (HK-2) comprising
3,5-dihydroxy-4-methoxybenzyl alcohol as an active ingredient, the elevating agent having an action of elevating basal respiration, maximal respiration, and ATP production of intracellular mitochondria in a human renal proximal tubular cell (HK-2).
